# EUROPÄISCHE PATENTANMELDUNG

(11) **EP 2 567 734 A2**
(43) Veröffentlichungstag der Anmeldung: **13.03.2013**
(21) Anmeldenummer: 12183177.0
(22) Anmeldetag: 05.09.2012
(51) Int. Cl.: A61Q 11/00, A61K 8/97

(54) **Substanz für die Zubereitung eines Mundspülmittels**

(30) Priorität: 06.09.2011 DE 102011053297
(71) Anmelder: YesVital GmbH, 83135 Schechen (DE)
(72) Erfinder: Krug, Stefanija, 83135 Schechen (DE)
(74) Vertreter: Bernhardt, Reinhold

(57) **Zusammenfassung**

Die Erfindung betrifft eine Substanz, insbesondere Trockensubstanz, für die Zubereitung eines Mundspülmittels zur Bekämpfung von Schädigungen des Mund- und Rachenraums, insbesondere von Mukositis, Stomatitis, Gingivitis, Aphten, Parodontitis, der Hand-Fuß-Mund-Krankheit und/oder Soor. Erfindungsgemäß bilden zerkleinerte Weintraubenkerne einen wirksamen Bestandteil dieser Substanz.

## Beschreibung

Die Erfindung betrifft eine Substanz, insbesondere Trockensubstanz, für die Zubereitung eines Mundspülmittels zur Bekämpfung von Schädigungen des Mund- und Rachenraums.

Aus der DE 100 06 837A1 ist es bekannt, Weintraubenkerne, die in den beim Keltern anfallenden Maischerückständen enthalten sind, zur Herstellung von Nahrungsergänzungs- und Hauptpflegemitteln zu nutzen.

Durch die vorliegende Erfindung werden weitere Verwertungsmöglichkeiten für Weintraubenkerne erschlossen.

Erfindungsgemäß weist die eingangs genannte Substanz für die Zubereitung eines Schädigungen des Mund- und Rachenraums bekämpfenden Mundspülmittels zerkleinerte Weintraubenkerne auf.

Insbesondere hat es sich herausgestellt, dass das mit Hilfe der erfindungsgemäßen Substanz zubereitete Mundspülmittel, zur Bekämpfung solcher Schädigungen des Mund- und Rachenraums, die als Folge einer Chemo- oder Strahlentherapie auftreten, vor allem Mukositis und Stomatitis, geeignet ist. Auch eine besondere Eignung zur Bekämpfung von Schädigungen des Mund- und Rachenraums infolge lang andauernder Behandlung mit Asthmaspray, insbesondere zur Bekämpfung der Gingivitis, wurde festgestellt.
Das durch die erfindungsgemäße Substanz zubereitete Mundspülmittel kann ferner zur Bekämpfung von Aphten, Parodontitis, der Hand-Fuß-Mund-Krankheit und/oder Soor eingesetzt werden.

In einer bevorzugten Ausführungsform der Erfindung ist die zerkleinerte Weintraubenkerne enthaltende Substanz zur Zubereitung des Mundspülmittels unter Zugabe von Wasser, als zur Zubereitung eines Mundwassers, vorgesehen.

Vorzugsweise handelt es sich bei der Substanz um eine Trockensubstanz, auf die zur Zubereitung des Mundspülmittels, insbesondere heißes Wasser, aufzugießen ist.

In einer zweckmäßigen Ausführungsform ist die Substanz daher in einem Aufgussbeutel enthalten.

Bei der Substanz handelt es sich vorzugsweise um eine Mischung, die neben den zerkleinerten Traubenkernen ein getrocknetes Kraut oder mehrere solche Kräuter aufweist.

Bei dem Kraut handelt es sich zweckmäßig um ein geschmacksbildendes Teekraut oder/und ein die therapeutische Wirkung unterstützendes Heilkraut, das insbesondere in Kombination mit den zerkleinerten Traubenkernen wirksam ist.

Vorzugsweise sind die Weintraubenkerne zu einem feinen Pulver zermalen, vorzugsweise mit einer mittleren Korngrößer < 100 µm.

Zweckmäßig werden die Traubenkerne wenigstens teilweise entölt, z.B. durch Pressen, so dass das daraus hergestellte Traubenkernpulver dauerhaft haltbar ist.

Der Anteil der zermahlenen Traubenkerne in der Mischung liegt vorzugsweise zwischen 40 und 70 Gew.-%.

Die genannte Mischung kann neben Kräutern ferner süßende oder geschmacksbildende Komponenten enthalten.

Die Erfindung wird nachfolgend anhand von Beispielen weiter erläutert.

### Beispiel 1

Aus Maische separierte Traubenkerne wurden nach dem in der hier einbezogenen DE 10 006 837A1 beschriebenen Verfahren teilweise entölt und zu einem feinen Pulver mit Korngrößen < 100 pm zermahlen.

Dem Pulver wurden getrocknete und zerkleinerte Kräuter zugesetzt, im vorliegenden Fall Gänsefingerkraut, Salbei, Ringelblumenkraut und Lavendel. Der Anteil des Traubenkernpulvers betrug 60 Gew.-%.

Zur Herstellung von Mundwasserproben wurde jeweils auf 3 g dieser Mischung 0,3 Liter heißes Wasser mit einer Temperatur knapp unter 100°C aufgegossen.

Nach einer Einwirk-, Klär- und Abkühldauer von 10 bis 30 Min. wurden die Mundwasserproben insgesamt zwanzig unter Mukositis leidenden einer Chemo- oder/und Strahlentherapie ausgesetzten Krebspatienten zur Mundspülung verabreicht. Bei mindestens drei Anwendungen täglich lag die Spüldauer bei jeweils vollständigem Verbrauch der Probe zwischen 5 und 10 Min., wobei die aufgenommene Spülmenge jeweils nach der Spülung ausgespien wurde.

Bei allen Patienten stellte sich schon nach zwei bis drei Tagen eine deutliche Besserung ein. Nach fünf bis sieben Tagen waren alle Patienten beschwerdefrei.

Erkennbar wirkt das Mundspülmittel in zwei Stufen, indem es zunächst für eine schnelle Ablösung beschädigter Teile der Mund- und Rachenschleimhaut sorgt und in einer zweiten Phase eine schnelle Regenerierung dieser Schleimhäute bewirkt.

### Beispiel 2

Abweichend von Beispiel 1 wurden die Mengen der das Traubenkernpulver enthaltenden Trockensubstanz bei gleicher Aufgussmenge an Wasser einmal halbiert und einmal verdoppelt.
Je zwei von vier Patienten mit Mukositis wurden mit der halben und doppelten Menge behandelt. Gegenüber der im Beispiel 1 ermittelten Abklingzeit der Beschwerden wurde bei doppelter Menge an Trockensubstanz keine weitere Verkürzung festgestellt. Bei halber Menge an Trockensubstanz trat jedoch eine merkliche Wirkungsminderung unter deutlicher Verlängerung der Abklingzeit ein.

### Beispiel 3

Gemäß Beispiel 1 wurde einzelnen Patienten mit Aphten, Parodontitis, der Hand-Fuß-Mund-Krankheit, Soor und durch Asthmaspray hervorgerufener Gingivitis Spülproben verabreicht. In allen Fällen nahmen die Beschwerden nach Behandlungsdauer von rund einer Woche deutlich erkennbar ab.

## Patentansprüche

1. Substanz, insbesondere Trockensubstanz, für die Zubereitung eines Mundspülmittels zur Bekämpfung von Schädigungen des Mund- und Rachenraums,
**dadurch gekennzeichnet,**
**dass** die Substanz zerkleinerte Weintraubenkerne umfasst.

2. Substanz nach Anspruch 1,
**dadurch gekennzeichnet,**
**dass** das Mundspülmittel zur Bekämpfung solcher Schädigungen infolge Chemo- oder Strahlentherapie, insbesondere Mukositis und Stomatitis, und durch Asthmaspray, insbesondere Gingivitis, sowie zur Bekämpfung von Aphten, Parodontitis, der Hand-Fuß-Mund-Krankheit oder/und Soor zubereitet ist.

3. Substanz nach einem der Ansprüche 1 bis 2,
**dadurch gekennzeichnet,**
**dass** die Substanz zur Zubereitung eines Mundwassers vorgesehen ist.

4. Substanz nach einem der Ansprüche 1 bis 3,
**dadurch gekennzeichnet,**
**dass** die Substanz zur Zubereitung des Mundspülmittels durch Aufgießen von, vorzugsweise heißem, Wasser vorgesehen ist.

5. Substanz nach einem der Ansprüche 1 bis 4,
**dadurch gekennzeichnet,**
**dass** die Substanz in einem Aufgussbeutel enthalten ist.

6. Substanz nach einem der Ansprüche 1 bis 5,
**dadurch gekennzeichnet,**
**dass** die Substanz eine Mischung ist, die neben den zerkleinerten Weintraubenkernen wenigstens ein getrocknetes Kraut aufweist.

7. Substanz nach Anspruch 6,
**dadurch gekennzeichnet,**
**dass** das getrocknete Kraut ein geschmacksbildendes Kraut, vorzugsweise ein Teekraut, oder/und eine Heilkraut ist.

8. Substanz nach einem der Ansprüche 1 bis 7,
**dadurch gekennzeichnet,**
**dass** die zu zerkleinerten Weintraubenkerne wenigstens teilweise, vorzugsweise durch Pressen, entölt sind.

9. Substanz nach einem der Ansprüche 1 bis 8,
**dadurch gekennzeichnet,**
**dass** die Weintraubenkerne zu einem Pulver, vorzugsweise mit einer mittleren Korngröße kleiner 100 µm, zerkleinert sind.

10. Substanz nach einem der Ansprüche 6 bis 9,
**dadurch gekennzeichnet,**
**dass** der Anteil der zerkleinerten Traubenkerne in der Mischung zwischen 40 und 70 Gew.-% beträgt.

11. Substanz nach einem der Ansprüche 1 bis 10,
**dadurch gekennzeichnet,**
**dass** die Mischung neben dem den zerkleinertem Traubenkernen und dem Kraut ferner eine süßende oder/und geschmacksbildende Komponente enthält.

12. Substanz nach einem der Ansprüche 1 bis 11,
**dadurch gekennzeichnet,**
**dass** das Mundwasser durch Aufgießen von 0,3 Liter Wasser je 3 g der Trockensubstanz hergestellt ist.
